(19) Europäisches Patentamt / European Patent Office / Office européen des brevets

(11) **EP 3 864 624 B1**

(12) **FASCICULE DE BREVET EUROPEEN**

(45) Date de publication et mention de la délivrance du brevet:
**23.11.2022 Bulletin 2022/47**

(21) Numéro de dépôt: **19816370.1**

(22) Date de dépôt: **07.10.2019**

(51) Classification Internationale des Brevets (IPC):
**G06T 11/00** (2006.01)    **G01T 1/36** (2006.01)
**G01T 1/29** (2006.01)    **A61B 6/00** (2006.01)
**G01N 23/04** (2018.01)    **G06T 5/00** (2006.01)
**G01N 23/046** (2018.01)

(52) Classification Coopérative des Brevets (CPC):
**G06T 11/005; A61B 6/482; A61B 6/5282;
G01N 23/046; G01T 1/295; G01T 1/36;
G06T 5/009;** G06T 2207/10081; G06T 2207/10116

(86) Numéro de dépôt international:
**PCT/FR2019/052376**

(87) Numéro de publication internationale:
**WO 2020/074820 (16.04.2020 Gazette 2020/16)**

(54) **PROCÉDÉ DE CORRECTION D'UNE IMAGE SPECTRALE**

VERFAHREN ZUR KORREKTUR EINES SPEKTRALBILDES

METHOD FOR CORRECTING A SPECTRAL IMAGE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.10.2018 FR 1859368**

(43) Date de publication de la demande:
**18.08.2021 Bulletin 2021/33**

(73) Titulaires:
• **COMMISSARIAT À L'ÉNERGIE ATOMIQUE ET AUX
ÉNERGIES ALTERNATIVES**
**75015 Paris (FR)**
• **Centre national de la recherche scientifique**
**75016 Paris (FR)**
• **Institut National des Sciences Appliquees
de Lyon (Insa Lyon)**
**69100 Villeurbanne (FR)**

(72) Inventeurs:
• **PIVOT, Odran**
**38054 Cedex 09 GRENOBLE (FR)**
• **TABARY, Joachim**
**38054 Cedex 09 GRENOBLE (FR)**
• **FOURNIER, Clarisse**
**38054 Cedex 09 GRENOBLE (FR)**
• **LETANG, Jean Michel**
**69100 VILLEURBANNE (FR)**
• **RIT, Simon**
**69100 VILLEURBANNE (FR)**

(74) Mandataire: **INNOV-GROUP**
**310, avenue Berthelot**
**69372 Lyon Cedex 08 (FR)**

(56) Documents cités:
**EP-A1- 3 153 888**    **US-A1- 2007 242 794**
**US-A1- 2007 268 997**

• **SOSSIN ARTUR ET AL: "Characterizing the
behavior of scattered radiation in multi-energy
x-ray imaging", NUCLEAR INSTRUMENTS &
METHODS IN PHYSICS RESEARCH. SECTION A,
vol. 850, 18 janvier 2017 (2017-01-18), pages
25-34, XP029917295, ISSN: 0168-9002, DOI:
10.1016/J.NIMA.2017.01.032**

- ALEXANDER KATSEVICH ET AL: "High-Quality 3-D MicroCT Imaging of Source Rocks - Novel Methodology to Measure and Correct for X-Ray Scatter", PROCEEDINGS OF THE 6TH UNCONVENTIONAL RESOURCES TECHNOLOGY CONFERENCE, 1 janvier 2018 (2018-01-01), XP055612288, Tulsa, OK, USA DOI: 10.15530/urtec-2018-2902457 ISBN: 978-0-9912144-5-7

- ALEXANDER KATSEVICH ET AL: "High-Quality 3-D MicroCT Imaging of Source Rocks - Novel Methodology to Measure and Correct for X-Ray Scatter", PROCEEDINGS OF THE 6TH UNCONVENTIONAL RESOURCES TECHNOLOGY CONFERENCE,

**Description**

## DOMAINE TECHNIQUE

Le domaine technique de l'invention est la correction d'une image spectrale formée par un détecteur pixellisé. L'invention peut notamment s'appliquer dans le domaine de l'imagerie de rayons X ou gamma, par exemple dans le cadre de l'aide au diagnostic médical.

## ART ANTERIEUR

Le contrôle d'objets par rayonnement X, dans le domaine médical ou industriel, est très répandu. Les procédés existants consistent à disposer un objet entre une source de rayonnement et un détecteur, puis à irradier l'objet à l'aide de la source. Le détecteur forme alors une image, généralement en deux dimensions, du rayonnement transmis par l'objet. Cette image est représentative de l'atténuation, par l'objet, du rayonnement émis par la source.

Le rayonnement transmis par l'objet comporte généralement une composante résultant de la diffusion, par l'objet, du rayonnement émis par la source. Elle est d'autant plus significative que l'énergie du rayonnement est faible et/ou que l'objet est constitué de matériaux dont le numéro atomique est élevé. Cette composante, appelée communément rayonnement de diffusion, perturbe l'interprétation des images, car elle n'est qu'indirectement liée à l'atténuation par l'objet. De plus, alors que le rayonnement non diffusé, dit rayonnement primaire, se propage entre la source et le détecteur selon une trajectoire rectiligne, le rayonnement diffusé a pour origine un point quelconque de l'objet, et sa trajectoire, depuis ce point origine, est distribuée selon différents angles. On recherche donc à estimer cette composante de diffusion, de façon à l'extraire du signal mesuré par le détecteur, préalablement au traitement des images en vue de leur interprétation.

Des outils de simulation numérique permettent de déterminer le rayonnement primaire et le rayonnement de diffusion ayant traversé un objet analysé. Un exemple est donné dans la publication Sossin A. "Fast scattering simulation tool for multi-energy x-ray imaging" Nuclear Instruments and Methods in Physics Research, A 802 (2015) 60-66. De tels outils facilitent le développement de systèmes de radiographie ou de tomographie.

Dans le domaine de l'imagerie médicale, de nombreuses méthodes ont été développées pour tenter d'estimer et de réduire la part du rayonnement diffusé, de façon à obtenir une image essentiellement représentative du rayonnement non diffusé, dit rayonnement primaire, se propageant entre la source et l'objet selon une direction rectiligne. Par exemple, le brevet EP3153888 décrit un procédé au cours duquel un masque, définissant un damier, est disposé entre une source de rayonnement et un détecteur pixelisé. Deux acquisitions sont effectuées, respectivement avec et sans masque. Une comparaison entre les deux acquisitions permet d'estimer le spectre de la composante de diffusion du rayonnement détecté par chaque pixel du détecteur. Si un tel procédé s'avère efficace, il suppose une double acquisition, avec et sans masque, ce qui nécessite la conception d'un masque amovible. De plus, le fait d'effectuer une double acquisition peut sembler en contradiction avec la nécessité de réduction de la dosimétrie lors des examens médicaux.

Les inventeurs proposent un procédé alternatif permettant d'estimer le spectre d'un rayonnement diffusé par un objet irradié, et se propageant vers les pixels d'un détecteur spectrométrique. Le spectre ainsi estimé est utilisé pour corriger le spectre du rayonnement détecté par chaque pixel. Le procédé n'impose pas d'effectuer une double acquisition, avec et sans masque.

## EXPOSE DE L'INVENTION

Un premier objet de l'invention est un procédé de correction d'une image spectrale formée par un rayonnement électromagnétique ionisant transmis par un objet, l'objet étant disposé entre une source d'irradiation et un détecteur, la source d'irradiation étant apte à émettre un rayonnement électromagnétique ionisant, dit rayonnement incident, vers l'objet ;

- le détecteur comportant des pixels, chaque pixel étant configuré pour détecter un rayonnement transmis par l'objet vers le détecteur, et à en mesurer un spectre, le rayonnement transmis comportant un rayonnement diffusé, causé par la diffusion du rayonnement incident dans l'objet, et un rayonnement primaire;
- un masque étant interposé entre la source et le détecteur, le masque comportant des éléments absorbants, aptes à atténuer une partie du rayonnement incident, la projection de chaque élément absorbant sur le détecteur formant une zone d'ombre, de telle sorte que le détecteur comporte une pluralité de zones d'ombre espacées les unes des autres, chaque zone d'ombre comportant au moins un pixel;

le procédé comportant les étapes suivantes :

a) irradiation de l'objet et mesure, par les pixels du détecteur, d'un spectre en énergie, représentatif du rayonnement transmis par l'objet ;

b) définition d'un modèle spatial du spectre du rayonnement diffusé, de façon à obtenir, en différents pixels du détecteur, une estimation du spectre du rayonnement diffusé détecté par chaque pixel, le modèle spatial étant défini par des paramètres;

c) prise en compte d'une fonction de coût, la fonction de coût étant définie en différents pixels, et prenant en compte, au niveau de chaque pixel, une variation spatiale d'une estimation du spectre du rayonnement primaire transmis par l'objet en l'absence de masque, l'estimation du spectre du rayonnement primaire en l'absence de masque étant obtenue en comparant, pour chaque pixel:

- le spectre mesuré par le pixel lors de l'étape a) ;
- une estimation du spectre du rayonnement diffusé obtenue à l'aide du modèle spatial résultant de l'étape b) ;

d) détermination des paramètres du modèle spatial pour lesquels la fonction de coût est minimale ou maximale ou se rapproche d'une valeur prédéterminée ;

e) pour au moins un pixel, estimation du spectre rayonnement diffusé atteignant le pixel, en appliquant les paramètres du modèle spatial déterminés lors de l'étape d).

Le procédé peut comporter une étape :

f) pour au moins un pixel, correction du spectre mesuré lors de l'étape a) en utilisant l'estimation du spectre du rayonnement diffusé résultant de l'étape e), de façon à obtenir un spectre corrigé correspondant à une estimation du spectre du rayonnement primaire atteignant le pixel.

Lors de l'étape f), le spectre corrigé peut correspondre à une estimation du spectre du rayonnement primaire atteignant le pixel en l'absence de masque disposé entre la source et le détecteur, ce qui correspond une variante préférée, ou en présence du masque.

Par fonction définie en différents pixels, on entend une fonction établie à partir du spectre mesuré en différents pixels.

Par rayonnement transmis par l'objet, ou rayonnement atteignant un pixel, ou rayonnement se propageant vers le pixel, on entend un rayonnement incident au pixel ou un rayonnement détecté par le pixel. Le passage entre le rayonnement incident au pixel et le rayonnement détecté par le pixel est établi en prenant en compte une fonction de réponse du pixel. La fonction de réponse peut notamment être représentée sous une forme matricielle.

Le masque peut être disposé entre la source et l'objet. Alternativement, le masque peut être disposé entre l'objet et le détecteur.

Par spectre représentatif du rayonnement transmis par l'objet, on entend un spectre du rayonnement transmis par l'objet ou un spectre obtenu à partir du spectre du rayonnement transmis par l'objet. Il peut par exemple s'agir d'un spectre résultant d'une normalisation du spectre du rayonnement transmis par l'objet.

De préférence dans lequel les pixels du détecteur s'étendent selon un plan de détection ; le modèle spatial est alors défini au moins selon deux axes définissant le plan de détection. Selon un mode de réalisation :

- l'étape a) est mise en œuvre selon une pluralité de configurations, à chaque configuration étant associée une orientation du détecteur par rapport à l'objet, de façon à mesurer, par chaque pixel, des spectres correspondant à différentes orientations ;
- le modèle spatial défini lors de l'étape b) prend en compte une variation du spectre du rayonnement diffusé en fonction de l'orientation du détecteur par rapport à l'objet.

Lors de l'étape c), l'estimation du spectre primaire transmis par l'objet vers chaque pixel, en l'absence de masque, peut être obtenue en appliquant une matrice de correction, définie pour chaque pixel, à une différence entre :

- le spectre mesuré par le pixel lors de l'étape a) ;
- une estimation du spectre du rayonnement diffusé obtenue à l'aide du modèle paramétrique résultant de l'étape b).

La matrice de correction peut être obtenue au cours d'une phase de calibration, sans objet entre la source d'irradiation et le détecteur, selon les étapes de calibration suivantes :

cal-i) irradiation du détecteur respectivement en interposant et sans interposer le masque entre la source et le détecteur, de façon à obtenir, pour chaque pixel, un spectre de plein flux et un spectre de masque;

cal-ii) prise en compte d'une fonction paramétrique pour déterminer des termes de la matrice, la fonction paramétrique dépendant de paramètres;

cal-iii) prise en compte du spectre de plein flux et du spectre de masque pour déterminer les paramètres de la fonction paramétrique, de façon à déterminer les termes de la matrice de correction.

La matrice de correction est de préférence triangulaire.

Le modèle spatial du rayonnement de diffusion peut être exprimé selon un produit :

- d'une matrice représentative d'une répartition spatiale du rayonnement diffusé ;
- d'un vecteur de paramètres comportant les paramètres du modèle spatial de diffusion;

de telle sorte que l'étape d) comporte une estimation du vecteur de paramètres.

Selon un mode de réalisation, l'étape b) comporte une définition d'un modèle spectral du rayonnement diffusé, pour prendre en compte, en chaque pixel, une évolution spectrale du rayonnement diffusé, de telle sorte que le modèle spatial et le modèle spectral sont définis par un vecteur de paramètres. Selon ce mode de réalisation, le modèle spatial et le modèle spectral du rayonnement de diffusion peuvent être exprimés selon un produit :

- d'une matrice représentative d'une répartition spatiale du rayonnement diffusé ;
- d'une matrice représentative de l'évolution spectrale du rayonnement diffusé ;
- du vecteur de paramètres;

de telle sorte que l'étape d) comporte une estimation du vecteur de paramètres.

Chaque élément absorbant du masque est, de préférence, apte à absorber entre 5 % et 80 % du rayonnement auquel il est exposé, dans une bande d'énergie prédéterminée. Le masque s'étendant selon une surface, chaque élément absorbant est de préférence distant d'un autre élément absorbant d'une distance inférieure à 1 cm.

Selon un mode de réalisation, l'étape a) comporte une normalisation du spectre du rayonnement transmis par l'objet, mesuré par le pixel, par un spectre mesuré par le pixel sans objet disposé entre la source et le détecteur.

Le procédé peut être mis en œuvre en déterminant des pixels du détecteur considérés comme pertinents, les spectres mesurés par ces derniers faisant l'objet d'une surpondération dans le calcul de la fonction de coût. Les pixels pertinents peuvent être les pixels adjacents des zones d'ombre formées par le masque. Ils peuvent également être situés :

- en dehors des contours délimitant l'objet ;
- en dehors de structures internes de l'objet induisant un important contraste d'atténuation.

La fonction de coût peut comporter une matrice de pondération, dont les coefficients tendent à surpondérer une contribution des pixels pertinents relativement aux pixels non considérés comme pertinents.

Un deuxième objet de l'invention est un support d'enregistrement d'informations comportant des instructions pour l'exécution des étapes b) à e) ou b) à f) du procédé selon le premier objet de l'invention à partir des spectres acquis lors de l'étape a), ces instructions étant aptes à être exécutées par un microprocesseur.

Un troisième objet de l'invention est un dispositif pour l'acquisition de spectres d'un rayonnement transmis par un objet comportant :

- une source d'irradiation, configurée pour émettre un rayonnement électromagnétique ionisant, dit rayonnement incident, vers ledit objet;
- un détecteur comportant des pixels, chaque pixel étant configuré pour détecter un rayonnement transmis par l'objet vers le détecteur, et à en acquérir un spectre ;
- un masque, apte à être interposé entre la source et l'objet, ledit masque comportant des éléments absorbants, aptes à absorber une partie dudit rayonnement incident, et dont une projection sur le détecteur définit des zones d'ombres distantes les unes des autres ;
- un processeur, configuré pour recevoir des spectres acquis par chaque pixel, et à mettre en œuvre les étapes b) à e) ou b) à f) du procédé selon le premier objet de l'invention.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1A montre un dispositif permettant une mise en œuvre de l'invention.

La figure 1B représente un détail d'un masque faisant partie du dispositif.

La figure 1C illustre une rotation relative du détecteur et de la source par rapport à l'objet.

La figure 2 représente les principales étapes d'un mode de réalisation de l'invention.

La figure 3A représente une matrice de réponse du détecteur. Les figures 3B et 3C représentent respectivement une ligne et une colonne de cette matrice de réponse.

La figure 4A un exemple de matrice de correction établie pour un pixel. La figure 4B représente une ligne de la matrice de correction.

Les figures 5A, 5B et 5C représentent des résultats de modélisations simulant une image d'un objet modélisé, chaque image étant basée sur des spectres mesurés par les pixels d'un détecteur, ces spectres représentant respectivement le rayonnement total, le rayonnement primaire et l'estimation du rayonnement primaire selon l'invention. La figure 5D représente un profil horizontal obtenu à partir des figures 5A, 5B et 5C. La figure 5E montre des atténuations spectrales obtenues à partir du spectre détecté respectivement par le pixel central des figures 5A, 5B et 5C.

## EXPOSE DE MODES DE REALISATION PARTICULIERS

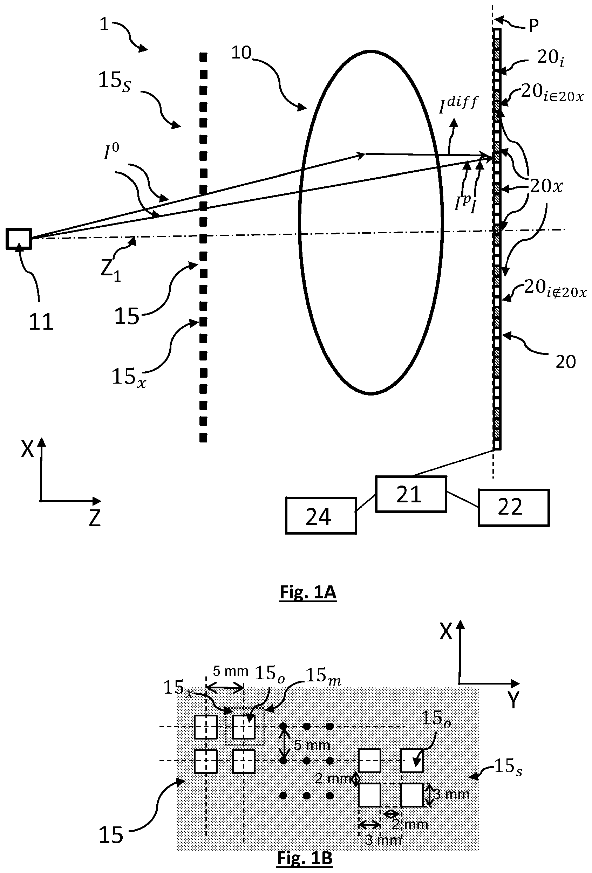

La figure 1A représente un mode de réalisation d'un dispositif 1 mettant en œuvre un procédé selon l'invention. Une source d'irradiation 11 émet un rayonnement électromagnétique ionisant $I^0$, dit rayonnement incident, vers un objet 10. L'objet 10 est disposé entre la source d'irradiation 11 et un détecteur de rayonnement 20. Le détecteur de rayonnement est un détecteur comprenant des pixels $20_i$ agencés selon un plan, dit plan de détection $P$. L'indice $i$ désigne une coordonnée de chaque pixel dans le plan de détection. Les pixels peuvent s'étendre selon une ligne, mais en général, ils s'étendent selon une matrice régulière bidimensionnelle. Dans ce cas, l'indice $i$ représente une coordonnée bidimensionnelle.

L'objet 10 peut être un tissu biologique vivant, par exemple une partie du corps d'un animal ou d'un être humain. Le dispositif est alors un dispositif d'imagerie médicale. L'objet peut également être une pièce industrielle ou un bagage, le dispositif étant alors utilisé à des fins de contrôle non destructif ou pour des contrôles liées à la sécurité.

Le terme rayonnement électromagnétique ionisant désigne un rayonnement électromagnétique constitué de photons d'énergie supérieure à 1 keV, et de préférence inférieure à 5 MeV. La plage d'énergie du rayonnement ionisant peut être comprise entre 1 keV et 2 MeV, mais elle s'étend le plus souvent entre 1 keV et 150 keV ou 300 keV. Le rayonnement ionisant peut être un rayonnement X ou $\gamma$. De préférence, la source de rayonnement ionisant est poly-énergétique, le rayonnement incident étant émis selon une plage d'énergie s'étendant généralement selon plusieurs dizaines voire centaines de keV. Il s'agit notamment d'un tube émetteur de rayons X.

Une partie des photons, constituant le rayonnement incident $I^0$, traversent l'objet et atteignent le détecteur 20, sans interagir avec l'objet. Ils se propagent vers le détecteur sans être déviés. Ces photons forment une composante primaire, ou rayonnement primaire $I^p$. D'autres photons constituant le rayonnement incident $I^0$ sont absorbés dans l'objet, par exemple par effet photoélectrique. Enfin, certains photons subissent une interaction de diffusion dans l'échantillon, de type diffusion inélastique Compton ou diffusion élastique Rayleigh. La diffusion, qu'elle soit inélastique ou élastique, engendre un changement de la direction du photon.

Ainsi, l'objet 10 irradié par la source 11 transmet au détecteur 20 un rayonnement $I$, dit rayonnement transmis, comportant :

- une composante directe, ou rayonnement primaire, $I^p$, n'ayant pas interagi avec l'objet, et dont la trajectoire depuis la source est rectiligne ;
- une composante de diffusion $I^{diff}$, ou rayonnement de diffusion, due à une diffusion du rayonnement incident dans l'objet.

Le rayonnement transmis $I$ par l'objet atteint les pixels du détecteur 20, chaque pixel détectant une partie de ce rayonnement. Le rayonnement transmis par l'objet et détecté par un pixel $20_i$ est noté $I_i$.

Comme évoqué en relation avec l'art antérieur, le rayonnement de diffusion $I^{diff}$ perturbe l'interprétation des mesures. En effet, contrairement au rayonnement primaire $I^p$, le rayonnement de diffusion se propage depuis l'objet vers le détecteur, selon une direction variable. Ainsi, une partie du rayonnement collecté par chaque pixel $20_i$ du détecteur ne provient non pas directement de la source de rayonnement 11, mais résulte du phénomène de diffusion. Or, l'interprétation des images est basée sur l'atténuation du rayonnement incident par le détecteur, cette dernière étant obtenue par un ratio, sur une plage d'énergie donnée, entre l'intensité du rayonnement primaire $I^p$ sur l'intensité du rayonnement incident $I^0$. Une bonne interprétation des images suppose la connaissance de l'intensité du rayonnement primaire $I^p$, alors que le rayonnement $I$ transmis par l'objet, et mesuré par le détecteur, comporte une somme dudit rayonnement primaire $I^p$ et du rayonnement diffusé $I^{diff}$.

Chaque pixel $20_i$ constitue un détecteur de rayonnement, comportant :

- un matériau détecteur, apte à interagir avec les photons du rayonnement $I$ transmis par l'objet, ce matériau étant de type scintillateur ou, de préférence, un matériau semiconducteur compatible avec une utilisation à la température ambiante, de type CdTe, CdZnTe ;
- un circuit électronique, apte à générer un signal dont l'amplitude dépend, et est de préférence proportionnelle, à l'énergie déposée par chaque photon interagissant dans le matériau détecteur ;
- un circuit de spectrométrie, apte à établir un spectre de l'énergie noté $S_i$ des signaux détectés pendant une période temporelle, dite période d'acquisition.

Ainsi, lorsque les pixels sont disposés régulièrement selon un agencement matriciel, chaque pixel est apte à produire un spectre $S_i$ du rayonnement transmis par l'objet. Le détecteur est alors apte à former plusieurs images, chaque image représentant un contenu de chaque spectre dans une plage d'énergie $\Delta E$ déterminée. Typiquement, chaque image comporte l'intégrale ou la valeur moyenne de chaque spectre $S_i$ dans ladite bande d'énergie. On parle alors d'imagerie spectrale, puisque le détecteur est à la fois résolu spatialement et spectralement.

Aussi, sous l'effet de l'irradiation par le rayonnement incident $I^0$, l'objet 10 transmet un rayonnement $I$, dit rayonnement transmis, vers un détecteur spectrométrique pixellisé 20, dont chaque pixel $20_i$ est apte à détecter ledit rayonnement transmis $I$ et à former un spectre en énergie $S_i$ du rayonnement $I_i$ ainsi détecté.

Le terme spectre en énergie correspond à un histogramme de l'amplitude des signaux détectés au cours d'une période d'acquisition du spectre. Une relation entre l'amplitude A et l'énergie $E$ peut être obtenue par une fonction d'étalonnage en énergie g telle que $E = g(A)$, selon des principes connus de l'homme du métier. Un spectre d'énergie $S_i$ est donc un vecteur, dont chaque terme $S_i(n)$ représente une quantité de rayonnement détecté par le pixel $20_i$ dans une plage d'énergie $E_n \pm \dfrac{\partial E}{2}$, avec $\partial E$ étant la largeur spectrale de chaque canal, $n$ désignant un numéro de canal.

A chaque canal $n$ correspond une énergie $E_n$, ou plus exactement une plage d'énergie $E_n \pm \dfrac{\partial E}{2}$.

Le spectre d'énergie $S_i$, mesuré par le pixel $20_i$, peut être normalisé par un spectre $S_{0,i}$ mesuré par le pixel $20_i$ en l'absence d'objet, ce dernier représentant le spectre du rayonnement $I^0$ émis par la source. Le spectre $S_{0,i}$ mesuré par le pixel en l'absence d'objet est communément désigné par le terme spectre plein flux. Ainsi, le spectre $S_i$ est le spectre mesuré par un pixel $20_i$, éventuellement normalisé par le spectre $S_{0,i}$. Le spectre $S_{0,i}$ est mesuré de préférence sans masque 15 disposé entre la source et le détecteur.

Chaque spectre en énergie $S_i$ peut être considéré comme une somme d'un spectre du rayonnement primaire, noté $S_i^p$ et d'un spectre du rayonnement de diffusion $S_i^{diff}$, à un terme de bruit près. Aussi, $S_i \approx S_i^p + S_i^{diff}$ (1). Le signe $\approx$ signifie une égalité à un terme de bruit près, ce bruit pouvant notamment résulter de la source d'irradiation, du détecteur ou d'effets dits d'empilement, se produisant lorsque deux photons incidents sont détectés simultanément.

Le dispositif comporte également un masque 15, disposé entre la source 11 et le détecteur 20 et dans cet exemple, entre la source 11 et l'objet 10, ce qui constitue la configuration préférée. Ce masque comporte des éléments absorbants $15_x$ répartis spatialement selon une surface $15_S$ selon laquelle s'étend le masque. Chaque élément absorbant est apte à atténuer partiellement une partie du rayonnement incident $I^0$ produit par la source d'irradiation. Les éléments absorbants sont répartis discrètement, de telle sorte que l'espace entre deux éléments absorbants adjacents est moins absorbant que lesdits éléments absorbants. Autrement dit, les éléments absorbants définissent une répartition spatiale discrète d'atténuations $att_{15}^x, att_{15}^{x'}$ de telle sorte qu'entre deux éléments absorbants adjacents $15_x$, $15_{x'}$, l'atténuation $att_{15}^0$ est inférieure à l'atténuation $att_{15}^x, att_{15}^{x'}$ associée à chaque élément absorbant.

Le terme atténuation est connu de l'homme du métier. Elle peut être exprimée selon l'expression $att_{15}^x(E) = -ln\left[\dfrac{I^x(E)}{I^0(E)}\right]$, où $I^0(E)$ désigne une intensité, à une énergie E, d'un rayonnement incident $I^0$ à un l'élément absorbant $15_x$ et $I^x(E)$ désigne une intensité, à ladite énergie E d'un rayonnement $I^x$ transmis par l'élément absorbant $15_x$.

L'atténuation peut être exprimée en fonction d'un coefficient d'atténuation linéaire $\mu(E)$, dépendant de l'énergie E et du matériau formant le masque, selon l'expression : $att_{15}^x(E) = -\mu(E)\ell_x$, $\ell_x$ désignant l'épaisseur de l'élément atténuant $15_x$ traversé.

D'une façon générale, l'interposition du masque 15 entre la source 11 et le détecteur 20 ne doit pas modifier significativement le rayonnement de diffusion parvenant au détecteur, par rapport à une configuration sans masque. Aussi, de préférence, chaque élément absorbant présente une atténuation, tel que précédemment défini, comprise entre 0.05 et 1.5, à une des énergies de la plage d'énergie selon laquelle est émis le rayonnement incident $I^0$, ou à l'énergie moyenne de cette plage d'énergie. Ainsi, en négligeant la diffusion, chaque élément absorbant atténue, de préférence, entre 5 % et 80% du rayonnement incident $I^0$ produit par la source et/ou traversant le masque dans l'espace s'étendant entre les éléments absorbants du masque. De préférence, l'atténuation est inférieure à 1 voire inférieure à 0.5 et de préférence inférieure à 0.3. Ainsi, chaque élément absorbant absorbe respectivement moins de 60 %, ou moins de 40%, et de préférence moins de 30% du rayonnement produit par la source, ou du rayonnement passant entre les éléments absorbants du masque. En dessous d'une atténuation égale à 0.05, correspondant à une atténuation de 5% du rayonnement produit par la source, les inventeurs considèrent que l'atténuation n'est pas suffisante. Autrement dit, le masque 15 permet donc d'établir un contraste d'atténuation, entre les éléments absorbants $15_x$ et l'espace s'étendant entre lesdits éléments absorbants, ces derniers absorbant entre 5% et 30%, voire 40%, voire davantage du rayonnement traversant ledit espace.

En complément ou alternativement, on peut définir une atténuation globale du masque 15 sous la forme d'un produit d'un facteur de remplissage par le pourcentage du rayonnement incident absorbé par le masque, ce dernier étant déterminé à une énergie de la plage d'énergie du rayonnement incident $I^0$ émis par la source d'irradiation 11, ou à une énergie moyenne de cette plage. Le facteur de remplissage correspond à un ratio entre la surface du masque occupée par l'ensemble des éléments absorbants $15_x$ et la surface totale du masque. L'atténuation globale du masque, ainsi définie, est de préférence supérieure à 0.5% et inférieure à 20%. Ainsi, un masque satisfaisant à cette condition peut avoir un facteur de remplissage égal à 0.2, chaque élément $15_x$ du masque absorbant 10% du rayonnement incident, ce qui donne une atténuation globale du masque, telle que précédemment définie, égale à 0.02 (2%).

Chaque élément absorbant peut avoir une forme quelconque, mais au moins une dimension, dans une direction de la surface $15_S$ selon lequel il s'étend, est inférieure à 5 mm, et de préférence inférieure à 2 mm voire à 1 mm. Dans l'ensemble des modes de réalisation précédemment décrits, le masque 15 s'étend de préférence selon un plan XY parallèle à un plan selon lequel s'étendent les pixels du détecteur.

L'espacement entre deux éléments absorbants adjacents, au niveau du masque, peut être inférieur à 5 mm, et est de préférence compris entre 1 mm et 5 mm. D'une façon générale, l'espacement entre deux éléments absorbants adjacents, après projection sur le détecteur 20, est avantageusement compris entre 1 et 10 cm, et de préférence inférieur à 5 cm ou à 3 cm. Comme décrit par la suite, la projection de chaque élément absorbant $15_x$ sur le détecteur définit une zone d'ombre élémentaire $20_x$. Chaque zone d'ombre élémentaire s'étend autour d'un point central. Avantageusement, l'espacement entre les points centraux de deux zones d'ombre élémentaires adjacentes est compris entre 1 et 10 cm, et de préférence compris entre 1cm et 5 cm. Par projection, on entend une projection selon la direction de propagation du rayonnement émis par la source.

Un exemple de masque est représenté sur la figure 1B. Dans cet exemple, le masque présente un arrangement régulier d'ouvertures carrées $15_O$ pratiquées dans une plaque absorbante. Le masque 15 forme une grille, comportant un agencement régulier de maille, chaque maille $15_m$ s'étendant autour d'une ouverture $15_O$. Sur la figure 1B, on a représenté une maille $15_m$ par un contour en pointillés. Le masque comporte des éléments absorbants $15_x$, chaque élément absorbant représentant le contour d'une ouverture 150, à l'intérieur d'une maille $15_m$. Dans cet exemple, chaque ouverture $15_O$ est un plot parallélépipédique, dont l'aire, selon la surface $15_S$ selon laquelle s'étend le masque, est de 3 mm * 3 mm, l'espacement entre le centre de chaque ouverture $15_O$ étant respectivement de 5 mm selon une première direction Y et selon une deuxième direction X perpendiculaire à la première direction. Dans cet exemple, le matériau constituant le masque est du graphite, d'épaisseur 5 mm. L'épaisseur s'entend selon une direction perpendiculaire à la surface selon laquelle s'étend le masque. D'une façon générale, le masque est réalisé selon un matériau suffisamment absorbant pour atténuer une portion suffisante du rayonnement primaire. On évite toutefois les matériaux trop denses, de type métaux lourds, par exemple le plomb, susceptibles de produire une diffusion significative du rayonnement incident avant que ce dernier n'atteigne l'objet. Parmi les matériaux préférés composant le masque, on peut citer, outre le graphite, l'aluminium, le cuivre, ou le bore.

D'autres géométries sont envisageables, en considérant par exemple des plots denses séparés par des espaces libres, tels que décrits dans EP3153888. On peut également envisager un espacement non régulier entre les différents éléments absorbants, ou une géométrie non régulière de chaque élément absorbant.

Les ouvertures $15_O$ pratiquées dans le masque 15 sont formées d'un matériau considéré comme transparent à l'égard des photons, dans la plage d'énergie considérée. Il s'agit par exemple d'air. Il peut également s'agir d'une fine épaisseur d'un plastique, d'un papier ou d'un métal léger, de type aluminium, fer, cuivre. Ainsi, au niveau de chaque ouverture $15_O$, le coefficient d'atténuation, tel que précédemment défini, est de préférence inférieur à 0.5, voire à 0.2 ou encore 0.1, et de façon plus générale suffisamment faible pour être négligé.

Le nombre d'ouvertures $15_O$ est dimensionné de façon à couvrir le champ d'observation du détecteur.

Le masque 15 est interposé entre la source 11 et le détecteur 20. Sa projection, sur le détecteur, selon la direction de propagation du rayonnement incident $I^0$, définit des zones d'ombre $20_x$, chaque zone d'ombre rassemblant les pixels du détecteur $20_{i \in 20_x}$ alignés par rapport à chaque élément absorbant $15_x$, selon la direction de propagation du rayonnement atteignant le détecteur. Plus précisément, comme précédemment évoqué, la projection de chaque élément absorbant $15_x$, selon la direction de propagation du rayonnement incident, forme une zone d'ombre $20_x$ sur le détecteur. L'indice x désigne la zone d'ombre considérée. Les pixels $20_{i \notin 20_x}$ n'appartenant pas à une zone d'ombre $20_x$ reçoivent un rayonnement non atténué par les éléments absorbants $15_x$, tandis que chaque pixel $20_{i \in 20_x}$ appartenant à une zone d'ombre reçoit un rayonnement atténué par un élément absorbant $15_x$, ce dernier étant situé sur une ligne s'étendant entre ledit pixel et la source d'irradiation 11.

Le dispositif comprend également une unité de calcul, ou processeur 21, par exemple un microprocesseur, est apte à traiter chaque spectre $S_i$ mesuré par les pixels du détecteur. En particulier, le processeur est un microprocesseur relié à une mémoire programmable 22 dans laquelle est stockée une séquence d'instructions pour effectuer les opérations de traitement de spectres et de calculs décrites dans cette description. Ces instructions peuvent être sauvegardées sur un support d'enregistrement, lisible par le processeur, de type disque dur, CDROM ou autre type de mémoire. Le processeur peut être relié à une unité d'affichage 24, par exemple un écran.

Un objectif de l'invention est de corriger le spectre $S_i$ mesuré par chaque pixel $20_i$, de façon à réduire la composante de diffusion $S_i^{diff}$ dûe à l'objet 10 et d'établir un spectre corrigé $S_i^*$ tel que $S_i^* \approx S_i'^p$ où $S_i'^p$ correspond au spectre primaire atteignant le pixel en l'absence de masque 15 entre la source 11 et le détecteur 20. Autrement dit, le spectre corrigé $S_i^*$ correspond à une estimation $\widehat{S}_i'^p$ du spectre du rayonnement primaire atteignant le pixel $20_i$ sans le masque interposé entre le la source et le détecteur: $S_i^* = \hat{S}_i'^p$ .

De façon alternative, il est possible d'établir un spectre corrigé $S_i^*$ de telle sorte qu'il correspond à une estimation du spectre $\hat{S}_i^p$ primaire atteignant l'objet en présence du masque 15, mais cela ne représente généralement pas une alternative préférée.

Un objectif de l'invention est d'évaluer le spectre de diffusion $S_i^{diff}$ atteignant les différents pixels $20_i$ du capteur d'image 20. Dans l'exemple de réalisation décrit par la suite, le spectre de diffusion $S_i^{diff}$ est le spectre d'une composante de diffusion du spectre $S_i$ mesuré par chaque pixel. L'invention est basée sur une hypothèse selon laquelle le spectre de diffusion ne varie pas de façon discontinue le long du plan de détection P défini par les pixels. De ce fait, on peut établir a priori un modèle spatial de diffusion $mod_K$, paramétrique, modélisant une dispersion spatiale du spectre de diffusion le long du plan de détection $P$. $K$ correspond aux paramètres du modèle. Ce modèle peut être par exemple approximé par une fonction Spline, c'est-à-dire une fonction définie, par morceaux, par des polynômes. Le modèle est défini selon des dimensions spatiales, correspondant par exemple aux axes X et Y du plan de détection P, et une dimension spectrale, traduisant l'évolution de la valeur du spectre en fonction de l'énergie. L'application du modèle spatial permet une estimation $\hat{S}_{i,K}^{diff}$ du spectre de diffusion en chaque pixel $20_i$ L'établissement du modèle paramétrique, et son ajustement, est un élément important de l'invention. Un exemple est fourni dans la suite de la description.

Un autre élément important est la prise en compte d'une matrice de correction $C_i$, définie pour chaque pixel $20_i$, la matrice de correction étant représentative de l'atténuation du rayonnement incident par le masque 15. La matrice de correction permet d'établir une relation entre le spectre primaire $S_i^p$ détecté par un pixel $20_i$ et le spectre primaire $\hat{S}_i^{p'}$ qui serait détecté par le pixel $20_i$ en l'absence de masque. Ainsi, $\hat{S}_i^{p'} = C_i \times S_i^p$ ; où $\times$ désigne le produit matriciel. La matrice de correction $C_i$ prend en compte l'atténuation par chaque élément absorbant du masque, ainsi que les imperfections de détection du pixel $20_i$ Un exemple d'établissement de la matrice de correction $C_i$ est présenté dans la suite de la description.

Chaque pixel $20_i$ mesure un spectre $S_i$. De ce fait, compte tenu de (1), on peut estimer que le spectre primaire $S_i^p$ mesuré par chaque pixel $20_i$ est tel que :

$$S_i^p \approx S_i - S_i^{diff} \ (2).$$

Or, le spectre $S_i^{diff}$ peut être estimé par application du modèle spatial de diffusion $mod_K$, de façon à obtenir une estimation $\hat{S}_{i,K}^{diff}$. Aussi, à partir de (2), on peut établir que :

$$S_i^p \approx S_i - \hat{S}_{i,K}^{diff} \ (3).$$

L'application de la matrice d'atténuation $C_i$ au spectre primaire $S_i^p$ mesuré par le pixel permet d'obtenir une estimation du spectre primaire $\hat{S}_i^{p\prime}$ qui aurait été mesuré par chaque pixel $20_i$, en l'absence de masque, selon l'expression :

$$\hat{S}_i^{p\prime} = C_i \times (S_i - \hat{S}_{i,K}^{diff}) \ (4).$$

Le symbole ′ dans la notation $\hat{S}_i^{p\prime}$ signifie qu'il s'agit du spectre primaire estimé en l'absence de masque 15. Le spectre primaire $\hat{S}_i^{p\prime}$ qui serait mesuré par chaque pixel $20_i$ en l'absence de masque 15 correspond au spectre de la composante primaire $I_i^{p\prime}$ du rayonnement $I_i$ mesuré par le pixel, en l'absence de masque.

On estime que localement, c'est-à-dire au niveau du voisinage de chaque pixel $20_i$, en l'absence de masque, la composante primaire $I_i^{p\prime}$ ne subit pas de fluctuations importantes. Par voisinage d'un pixel, on entend les pixels adjacents audit pixel. Ainsi, en l'absence de masque 15, le gradient spatial $\nabla \hat{S}_i^{p\prime}$ du spectre $\hat{S}_i^{p\prime}$ est faible.

De ce fait, les paramètres K du modèle paramétrique de diffusion peuvent être déterminés en considérant une fonction de coût $\mathcal{F}(K)$ établie à l'aide du gradient spatial $\nabla \hat{S}_i^{p\prime}$. Les paramètres $K$ du modèle paramétrique de diffusion sont ceux qui minimisent la fonction de coût $\mathcal{F}(K)$. Autrement dit, les paramètres K du modèle sont ceux qui "effacent" la trace du masque sur la distribution spatiale du spectre $\hat{S}_i^{p\prime}$.

Selon un premier exemple, la fonction de coût correspond à la somme du gradient spatial $\nabla \hat{S}_i^{p\prime}$ sur un nombre $N_i$ de pixels et sur un nombre $N_n$ de canaux d'énergie, de telle sorte que :

$$\mathcal{F}(K) = \sum_{i,n}^{2N_i,N_n} f(\nabla \hat{S}_i^{p\prime}(n)) \ (5)$$

$$\text{Soit } \mathcal{F}(K) = \sum_{j=1}^{2N_iN_n} f\left(\nabla C \times (S - \hat{S}_K^{diff})\big|_j\right) \ (5')$$

Dans (5') :

- f est une fonction, correspondant par exemple à une norme. Il peut par exemple s'agir d'une norme de rang 1 ou une norme de rang 2. f peut être une fonction de Huber ou de Charbonnier, ce type de fonction pouvant être considérée comme approximant une norme d'ordre 1.
- ∇ désigne l'opérateur gradient spatial, successivement calculé selon deux directions orthogonales X et Y. Il peut être exprimé sous la forme d'une matrice de dimension ($2N_iN_n$ ; $N_iN_n$).

- *j* est un super indice, prenant en compte une coordonnée spatiale *i* d'un pixel, une coordonnée spectrale *n* et une direction de calcul du gradient (selon l'axe X ou selon l'axe Y).
- S est un vecteur, de dimension $N_i N_n$ formé par une concaténation des spectres mesurés $S_i$.
- $\hat{S}_K^{diff}$ est un vecteur, de dimension $N_i N_n$ formé par une concaténation des spectres de diffusion estimés $\hat{S}_{i,K}^{diff}$.
- *C* est une matrice de dimension ($N_i N_n$; $N_i N_n$) obtenue par concaténation de l'ensemble des matrices de correction $C_i$ définies pour chaque pixel $20_i$.

La minimisation de la fonction de coût $\mathcal{F}(K)$ permet d'obtenir les paramètres *K* du modèle spatial du rayonnement diffusé. Autrement dit :

$$K = \arg\min_K (\mathcal{F}(K)) \ (6)$$

D'autres expressions de fonctions de coût sont possibles, de telle sorte que les paramètres K du modèle minimisent ou maximisent la fonction de coût, où la font tendre vers une valeur prédéterminée.

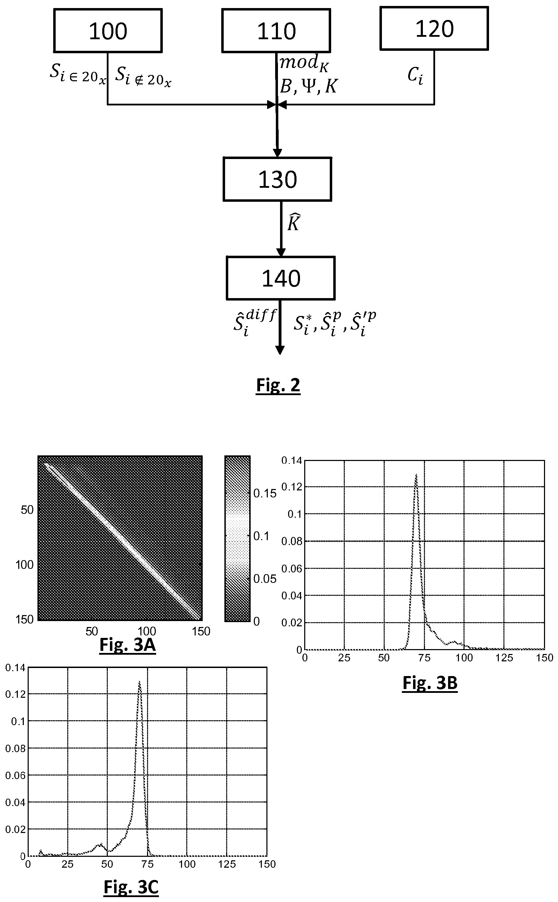

On va décrire, en lien avec la figure 2, les principales étapes d'un procédé selon l'invention.

Etape 100 : acquisition des spectres transmis par l'objet. Chaque pixel $20_{i \in 20_x}$ d'une zone d'ombre $20_x$ acquiert un spectre $S_{i \in 20_x}$, transmis par l'objet 10, en subissant une atténuation par un élément absorbant $15_x$. Chaque pixel $20_{i \notin 20_x}$ en dehors d'une zone d'ombre $20_x$ acquiert un spectre $S_{i \notin 20_x}$, transmis par l'objet 10, sans l'influence d'un élément absorbant $15_x$ du masque 15.

Etape 110 : établissement du modèle spatial de diffusion $mod_K$, conditionné par des paramètres *K*.

Le modèle spatial de diffusion comporte une composante spatiale, représentative de l'évolution spatiale du spectre de diffusion. Il peut également comporter une composante spectrale, représentative de l'évolution spectrale du spectre de diffusion.

En ce qui concerne la composante spatiale, elle peut être exprimée par une base B-splines à 2 dimensions spatiales, correspondant aux deux axes X et Y définissant le plan de détection. La base de B-splines peut être d'ordre 2, avec un écart entre deux nœuds de 32 pixels, les nœuds étant répartis selon les deux dimensions du plan de détection.

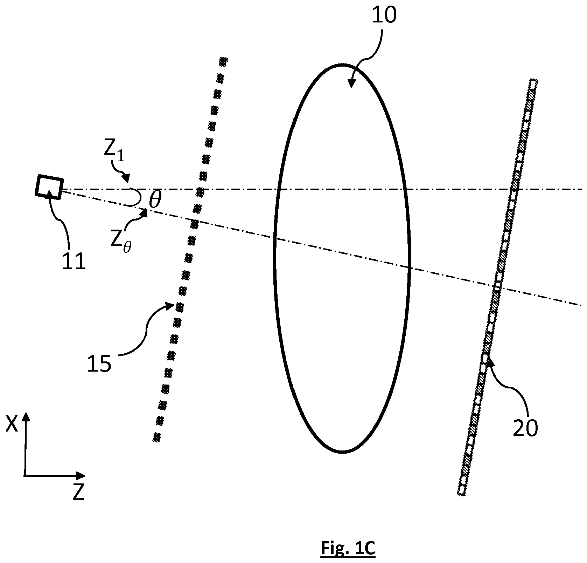

Selon une variante, décrite ultérieurement, on peut tenir compte d'une rotation de l'objet par rapport au détecteur selon plusieurs angles de rotation θ. Une telle configuration est représentée sur la figure 1C. Dans la configuration représentée sur la figure 1A, le détecteur 20 s'étend perpendiculairement à un axe $Z_1$, parallèle à l'axe Z. Dans la configuration représentée sur la figure 1C, le détecteur 20 s'étend perpendiculairement à un axe Ze. Entre les deux configurations respectivement représentées sur les figures 1A et 1C, le détecteur 20 et la source 11 ont subi une rotation d'angle θ par rapport à l'objet 10. Ce type de rotation est usuel dans les procédés de reconstruction tomographique de l'objet. Lorsque le détecteur est apte à subir une rotation par rapport à l'objet, le modèle spatial peut être exprimé selon une base B-splines à 3 dimensions (deux dimensions correspondant au plan de détection et une dimension correspondant à l'angle de rotation). D'une façon générale, le nombre de dimensions selon lequel est établi le modèle spatial correspond au nombre de degrés de liberté pris en compte pour modéliser le rayonnement de diffusion.

On peut définir un sous-modèle spectral, représentant la composante spectrale du modèle spatial. Dans notre exemple, le spectre mesuré $S_i$ subit un regroupement selon des groupes de canaux adjacents avant d'être traité par l'unité de traitement. Compte tenu des discontinuités pouvant apparaître entre des canaux ainsi regroupés, on prend en compte une base B-spline d'ordre 0, avec une distance inter-nœuds de 1 canal.

Les paramètres du modèle forment un vecteur de paramètres *K*, de dimension $N_K$, correspondant au nombre de nœuds du modèle.

Ainsi, le modèle paramétrique peut être exprimé sous la forme d'un produit matriciel, de telle sorte que

$$\hat{S}_{i,K}^{diff} = B \times \Psi \times K \ (10)$$

avec :

- *B* est une matrice représentant la composante spatiale du modèle spatial, de dimension [$N_i. N_n$; $N_{ki}. N_n$], . étant l'opérateur produit ;
- Ψ est une matrice représentant la composante spectrale (ou sous-modèle spectral) du modèle spatial, de dimension [$N_{ki}. N_n$; $N_k$] ; dans cet exemple, Ψ est la matrice identité. Cela signifie que le contenu des canaux d'énergie sont indépendants les uns des autres.
- K est un vecteur de paramètres comportant les paramètres du modèle, de dimension $N_k$ ;

- $N_{k_i}$ est le nombre de nœuds spatiaux considérés dans le modèle ;
- $\times$ est le produit matriciel.

Chaque terme de la matrice $B$ est tel que :

$$B[i.n; k_i.n] = \beta^2\left(\frac{u-u_{k_i}}{\Delta_u}\right)\beta^2\left(\frac{v-v_{k_i}}{\Delta_v}\right) \text{ (10')}$$

où :

- $u$ et $v$ désignent les coordonnées du pixel 20$i$ dans le plan du détecteur, respectivement selon les axes X et Y ;
- $u_{k_i}$ et $v_{k_i}$ désignent les coordonnées du nœud d'indice $k_i$ dans le plan du détecteur ;
- $\Delta_u$ et $\Delta_v$ sont les distances entre deux nœuds consécutifs respectivement l'axe X et l'axe Y.
- $\beta^2$ est une fonction de base B-spline, à l'ordre 2, avec :

$$\beta^2(w) = \frac{1}{2}\left(w^2 + 3w + \frac{9}{4}\right) \text{ si } w \in \left[-\frac{3}{2}, -\frac{1}{2}\right[ \text{ ;}$$

$$\beta^2(w) = \frac{9}{4} - w^2 \text{ si } w \in \left[-\frac{1}{2}, +\frac{1}{2}\right[ \text{ ;}$$

$$\beta^2(w) = \frac{1}{2}\left(w^2 - 3w + \frac{9}{4}\right) \text{ si } w \in \left[\frac{1}{2}, \frac{3}{2}\right[ \text{ ;}$$

$$\beta^2(w) = 0 \text{ pour } w < -\frac{3}{2} \text{ ou } w \geq \frac{3}{2}.$$

<u>Etape 120</u> : établissement d'une matrice de correction $C_i$ pour chaque pixel 20$_i$.

La prise en compte de l'atténuation par un élément absorbant 15$_x$ du masque 15 peut être effectuée par une matrice de correction $C_i$ définie pour chaque pixel 20$_i$. Pour les pixels 20$_{i \notin 20_x}$, la matrice de correction est unitaire. On décrit ci-après la matrice de correction $C_i$ pour des pixels 20$_{i \in 20_x}$ situés dans une zone d'ombre.

Selon une première approximation, basée sur une hypothèse d'un détecteur parfait, la matrice de correction peut être déterminée en construisant une matrice d'atténuation M, dont chaque terme correspondant à une atténuation dans une plage d'énergie donnée. Plus précisément, chaque terme

$$M(p,p) = e^{-\mu_x \ell_x} \text{ (11)}$$

- $\mu_x$ est le coefficient d'atténuation linéaire, dans la bande d'énergie $\Delta E_p$ associée à la ligne $p$ de la matrice d'atténuation M;
- $\ell_x$ est l'épaisseur de l'élément absorbant 15$_x$ dont la projection sur le masque 15 forme la zone d'ombre 20$_x$ considérée.

Ainsi, sans prise en compte de la réponse du détecteur, ou en considérant un détecteur parfait, $C_i = M^{-1}$.

Lorsque le détecteur n'est pas parfait, chaque pixel 20$_i$ peut être caractérisé par une matrice de réponse $D_i$, représentant les imperfections de la détection. Un exemple de la matrice de réponse, de taille $N \times M$, est représenté sur la figure 3A. $N$ est le nombre de canaux $n$ de chaque spectre formé par le détecteur et $M$ est le nombre de canaux selon lesquels le spectre incident au détecteur est discrétisé. $N$ et $M$ sont deux entiers positifs. Dans cet exemple, $N = M = 150$, mais de façon générale, le nombre de canaux du spectre est supérieur à 2, voire supérieur à 10, et peut atteindre plusieurs centaines. Chaque terme $D_i(u,v)$ de la matrice de réposne représente une probabilité qu'un photon incident au détecteur, d'énergie $v$, soit considéré par le détecteur comme ayant une énergie $u$.

Autrement dit, chaque ligne $D_i(u,\cdot)$ de la matrice, telle que celle représentée sur la figure 3B, représente une distribution de probabilité de l'énergie $v$ d'un photon atteignant le détecteur lorsqu'un photon d'énergie $u$ est détecté. La figure 3B représente la ligne 70 de la matrice $D_i$. De façon analogue, chaque colonne $D_i(\cdot, v)$ de la matrice, telle que celle représentée sur la figure 3C, correspond à une distribution de probabilité de l'énergie détectée $u$ par le détecteur lorsque le photon atteignant le détecteur a une énergie $v$. La figure 3C représente la colonne 70 de la matrice $D$. Plus la résolution en énergie du détecteur est fine, plus cette matrice tend vers une matrice diagonale. Dans le cas d'un détecteur parfait, la matrice $D_i$ est la matrice identité.

En prenant en compte les imperfections du détecteur, par le biais de la matrice de réponse $D_i$, la matrice de correction $C_i$ peut être exprimée comme suit :

$$C_i = D_i \times M^{-1} \times D_i^{-1} \quad (12)$$

Cela dit, la matrice de réponse $D_i$ n'est généralement pas inversible. Afin de prendre en compte la réponse de chaque pixel $20_i$ du détecteur, les inventeurs ont estimé que la matrice de correction $C_i$ est une matrice triangulaire, de dimension $(N_n, N_n)$, et dont chaque terme est tel que :

$$C_i(p, q) = \alpha_{i,p} h(p - q)\left[\delta\left(1 - \beta_{i,p}\right) - \beta_{i,p} e^{-\gamma_{i,p}(q-p)}\right] \quad (13);$$

où :

- $h$ est la fonction de Heaviside ;
- $\delta$ est la fonction de Dirac ;
- $\alpha_{i,p}$, $\beta_{i,p}$ et $\gamma_{i,p}$ sont des scalaires déterminés lors d'une phase de calibration. Ces scalaires sont associés à un pixel $20_i$.

La phase de calibration est réalisée en l'absence d'objet 10 entre le détecteur 20 et la source 11. Elle consiste à acquérir :

- une image spectrale, dite image plein flux $F^0$, sans masque 15 entre la source 11 et le détecteur 20 ;
- une image spectrale, dite image du masque $M^0$, en interposant le masque 15 entre la source 11 et le détecteur 20.

Pour chaque pixel $20_i$, les coefficients $\alpha_{i,p}$, $\beta_{i,p}$ et $\gamma_{i,p}$ sont tels que :

$$(\alpha_{i,p}, \beta_{i,p}, \gamma_{i,p}) = \underset{\alpha_{i,p}, \beta_{i,p}, \gamma_{i,p}}{\mathrm{argmin}} \left|F_i^0(p) - \sum_{q=1}^{N_n} C_i(p, q) M_i^0(q)\right| \quad (14)$$

où :

- $F_i^0(p)$ est la valeur du spectre plein flux $F_i^0$ mesuré par le pixel $20_i$ au canal d'énergie $p$.

- $M_i^0(q)$ est la valeur du spectre de masque $M_i^0$ mesuré par le pixel $20_i$ au canal d'énergie $q$.

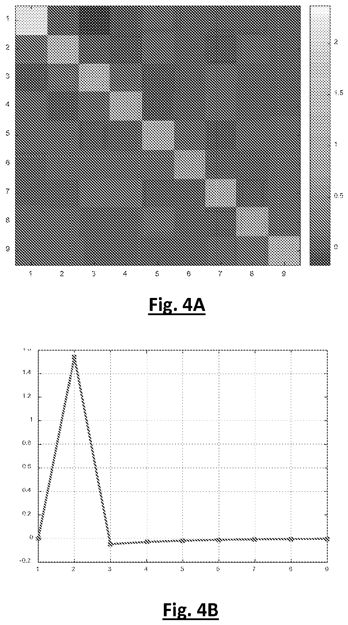

Les figures 4A et 4B montrent respectivement un exemple de matrice de correction ainsi qu'un profil de la deuxième ligne de cette matrice.

Etape 130 : estimation des paramètres $K$ du modèle.

Ayant défini le modèle spatial (étape 110), paramétré par le vecteur $K$, et après avoir mesuré le spectre du rayonnement transmis par l'objet, en chacun des pixels du détecteur (étape 100), on utilise les matrices de correction $C_i$ définies pour chaque pixel (étape 120) pour estimer les paramètres $K$ du modèle. Comme évoqué en lien avec les expressions (5) et (6), l'estimation des paramètres du modèle est effectué par une minimisation d'une fonction de coût. La fonction de coût peut être, par exemple :

$$\mathcal{F}(K) = \sum_{i,n}^{2N_i, N_n} f(\nabla \hat{S}_i^{p\prime}(n)) = \sum_{j=1}^{2N_i N_n} f\left(\left[\nabla C \times (S - B \times \Psi \times K)\right]\big|_j\right) \quad (15)$$

$C$ et $S$ correspondent à des matrices telles que définies en lien avec l'expression (5'). $j$ est le superindice défini en lien avec l'expression (5).

La fonction $f$ peut être une fonction de Charbonnier, avec $f(q) = \lambda^2 \sqrt{\left(1 + \left(\frac{q}{\lambda}\right)^2\right)} - 1$ , (16), où $\lambda^2$ est un paramètre scalaire, avec par exemple $\lambda = 10^{25}$.

On estime ainsi le vecteur $\hat{K}$ tel que $\hat{K} = \arg\min_{K} \mathcal{F}(K)$ . K

<u>Etape 140</u> : correction du spectre mesuré par chaque pixel.

L'estimation du vecteur $\hat{K}$ permet d'estimer le spectre de diffusion $\hat{S}_{i,\hat{K}}^{diff}$ , avec :

$$\hat{S}_{i,\hat{K}}^{diff} = B \times \Psi \times \hat{K} \ (17)$$

Le spectre $S_i$ mesuré par chaque pixel peut être corrigé de façon à obtenir un spectre corrigé $S_i^*$ . Ce dernier correspond à une estimation du spectre du rayonnement primaire $\hat{S}_i^p$ transmis par l'objet et détecté par chaque pixel, de telle sorte que :

$$S_i^* = \hat{S}_i^p = S_i - \hat{S}_i^{diff} \ (18), \text{ avec } \hat{S}_i^{diff} = \hat{S}_{i,\hat{K}}^{diff},$$

ou, de façon préférée :

$$S_i^* = \hat{S}_i^{'p} = C_i \times \left( S_i - \hat{S}_i^{diff} \right) \ (18').$$

Selon une variante, la position relative du détecteur par rapport à l'objet varie. Par exemple, l'ensemble, formé par la source d'irradiation 11 et le détecteur 20, tourne autour de l'objet, comme représenté sur la figure 1C. A chaque position du détecteur 20 par rapport à l'objet 10 correspond un angle θ. Le modèle spatial de diffusion peut prendre en compte une variabilité angulaire du spectre de diffusion, sous la forme d'un troisième axe spatial représentant l'angle θ. Si $N_\theta$ représente le nombre de pas angulaires considérés, le modèle spatial est tel que :

$$\hat{S}_{i,K}^{diff} = B \times \Psi \times K \ (10)$$

avec :

- $B$ est une matrice représentant le sous-modèle spatial, de dimension $[N_i. N_n. N_\theta; N_{k_i}. N_n. N_{k_\theta}]$ ;
- $k_\theta$ est le nombre de nœuds angulaires considérés ;
- . étant l'opérateur produit ;
- $\Psi$ est une matrice représentant le sous-modèle spectral, de dimension $[N_{k_i}. N_n. N_{k_\theta}; N_k]$;
- $K$ est un vecteur comportant les paramètres du modèle, de dimension $N_k$.

$$B[i.n.\theta; k_i.n.k_\theta] = \beta^2(\frac{u - u_{k_i}}{\Delta_u})\beta^2(\frac{v - v_{k_i}}{\Delta_v})\beta^2(\frac{\theta - \theta_{k_\theta}}{\Delta_\theta})$$

où :

- $u$ et $v$ désignent les coordonnées du pixel 20$i$ dans le plan du détecteur, respectivement selon les axes X et Y ;
- $u_{k_i}$ et $v_{k_i}$ désignent les coordonnées du nœud d'indice $k_i$ dans le plan du détecteur ;
- $\theta_{k_\theta}$ désigne la coordonnée angulaire du nœud d'indice $k_\theta$
- $\Delta_u$ et $\Delta_v$ sont les distances entre deux nœuds consécutifs respectivement l'axe X et l'axe Y;
- $\Delta_\theta$ est l'écart entre deux nœuds angulaires consécutifs ;
- $\beta^2$ est une fonction de base B-spline, à l'ordre 2.

L'expression (15) devient :

$$\mathcal{F}(K) = \sum_{i,n,\theta}^{2N_i,N_n,N_\theta} f(\nabla \hat{S}_{i,\theta}^{p\prime}(n)) = \sum_{j=1}^{2N_i N_n N_\theta} f\left([\nabla C \times (S - B \times \Psi \times K)]|_j\right) \text{ (19)}$$

Un tel mode de réalisation est pertinent lors d'acquisitions d'images en vue d'une reconstruction tomographique. Le spectre $S_{i,\theta}$, correspondant à chaque angle $\theta$, peut ainsi être corrigé en fonction d'une estimation, à chaque angle, du spectre de diffusion, de la même façon qu'explicité en lien avec les expressions (18) et (18').

Les procédés tels que décrits ci-avant peuvent être optimisés en privilégiant l'information issue de pixels pertinents, ou en se limitant à ces derniers, les pixels pertinents étant ceux pour lesquels la mise en œuvre de l'algorithme de minimisation est la plus efficace.

Les pixels pertinents peuvent être les pixels adjacents des frontières délimitant les zones d'ombre $20_x$ définies par le masque 15. Les pixels pertinents peuvent être déterminés à partir d'un gradient bidimensionnel de l'image du masque $M^0$ : ils correspondent aux composantes non nulles du gradient de l'image du masque. On peut établir une matrice de pondération W, telle que $W = diag(\nabla M^0)$.

Lorsqu'on ne prend pas en compte la variation angulaire selon $\theta$, $W$ est une matrice de dimension $2N_iN_n$; $2N_iN_n$ . Lorsqu'on prend en compte la variation angulaire, $W$ est une matrice de dimension $2N_iN_nN_\theta$; $2N_iN_nN_\theta$.

La matrice de pondération $W$ est prise en compte dans la détermination de la fonction de coût, de telle sorte que :

$$\mathcal{F}(K) = \sum_{j=1}^{2N_i N_n} f\left([W\nabla C \times (S - B \times \Psi \times K)]|_j\right) \text{ (20)}$$

ou

$$\mathcal{F}(K) = \sum_{j=1}^{2N_i N_n N_\theta} f\left([W\nabla C \times (S - B \times \Psi \times K)]|_j\right) \text{ (20')}$$

De préférence, les pixels pertinents ne doivent pas être situés sur les bords de l'objet 10, ou au niveau de structures internes de l'objet induisant un contraste d'atténuation important. En effet, au niveau des bordures de l'objet, ou lorsque l'objet présente de forts gradients locaux de l'atténuation, le spectre primaire transmis par l'objet subit des discontinuités. L'hypothèse d'une continuité locale du spectre primaire, prise en compte dans l'algorithme qui précède, n'est alors pas pertinente. Les pixels situés face aux bordures de l'objet ou face à d'importants gradients locaux d'atténuation sont donc considérés comme non pertinents et ne sont pas pris en compte dans l'établissement de la fonction de coût et lors de sa minimisation. Alternativement, les pixels considérés comme non pertinents peuvent être affectés d'un coefficient de pondération faible, de telle sorte que la fonction de coût dépend essentiellement des pixels considérés comme pertinents.

Essais

Des simulations ont été réalisées en considérant une source d'irradiation 11 sous la forme d'un tube à rayons X avec une anode de tungstène, soumis à une tension de 120 kV, filtrée par une épaisseur de 0.25 mm de cuivre et de 8.4 mm d'aluminium. Le détecteur 20 comprend 1024 (selon l'axe X) * 1024 pixels (selon l'axe Y), chaque pixel s'étendant selon une surface de 400 $\mu$m $\times$ 400 $\mu$m et selon une épaisseur de 3 mm. Le détecteur est résolu en énergie, et chaque pixel permet d'obtenir des spectres de 256 canaux d'énergie. Les pixels sont regroupés spatialement en groupes de 16 pixels adjacents (bining 4 $\times$ 4). Le spectre acquis par chaque groupe de pixel fait l'objet d'un regroupement en énergie de façon à former 9 canaux d'énergie, de largeur spectrale 10 keV, s'étendant entre 30 et 120 keV. Le masque utilisé est celui représenté sur la figure 1B.

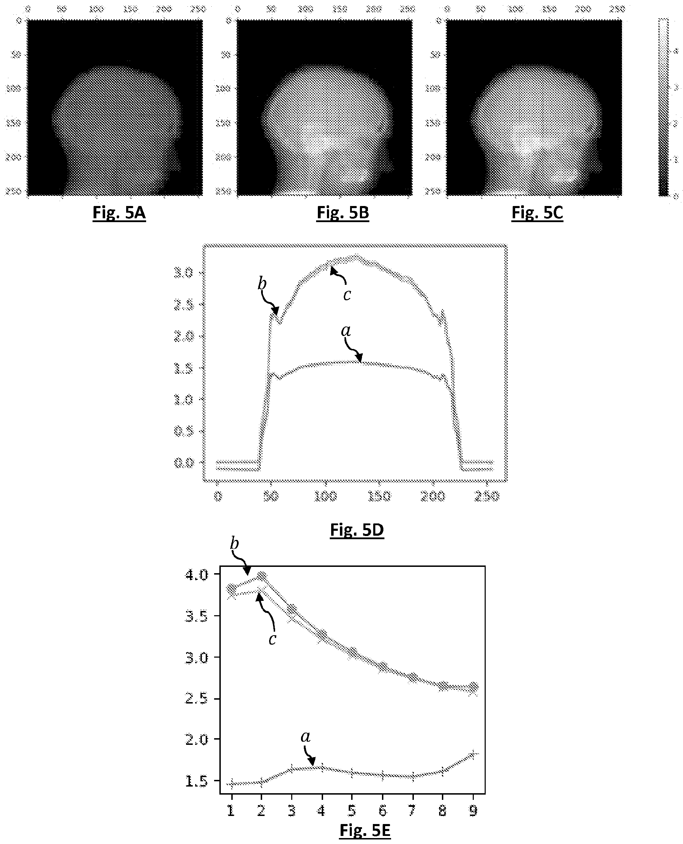

Les figures 5A, 5B et 5C montrent respectivement des images simulées d'un crâne. Elles représentent respectivement une distribution spatiale de l'atténuation, cette dernière étant obtenue à partir :

- du spectre $S_i$ détecté par chaque pixel ;

- du spectre primaire de référence $S_i^{p,ref}$ atteignant chaque pixel. Le spectre primaire de référence est obtenu par modélisation.

- du spectre corrigé $S_i^*$ selon l'invention. Le spectre corrigé correspond à une estimation du spectre primaire $\hat{S}_i^{\prime p}$ du rayonnement transmis par l'objet en l'absence de masque. Plus le spectre primaire estimé $\hat{S}_i^{\prime p}$ se rapproche du spectre primaire de référence $S_i^{p,ref}$, meilleure est la correction.

Dans chacun de ces cas, l'atténuation a été obtenue, en chaque pixel, en effectuant l'intégrale, selon les canaux d'énergie, du spectre considéré. Ainsi, l'atténuation représentée sur les figures 5A, 5B et 5C correspond respectivement

à $-ln\left(\frac{\sum_{n=1}^{Nn} S_i(n)}{\sum_{n=1}^{Nn} S_{i,0}(n)}\right)$, $-ln\left(\frac{\sum_{n=1}^{Nn} S_i^{p,ref}(n)}{\sum_{n=1}^{Nn} S_{i,0}(n)}\right)$ et $-ln\left(\frac{\sum_{n=1}^{Nn} \hat{S}_i^{'p}(n)}{\sum_{n=1}^{Nn} S_{i,0}(n)}\right)$. $S_{i,0}$ correspond au spectre, mesuré par un pixel

$20_i$, d'un rayonnement $I_0$ atteignant le détecteur en l'absence d'objet

Sur la figure 5A, le spectre considéré pour estimer l'atténuation comporte une forte composante d'un rayonnement de diffusion. Il en résulte une sous-estimation de l'atténuation. Les figures 5B et 5C sont cohérentes, et montrent une atténuation plus élevée que sur la figure 5A. Cela est dû au fait que l'atténuation a été estimée uniquement sur la base d'une estimation du spectre primaire, c'est-à-dire du spectre du rayonnement primaire transmis par l'objet et atteignant le détecteur.

La figure 5D montre des profils de l'atténuation le long d'une ligne horizontale représentée sur les figures 5A, 5B et 5C. Les spectres *a*, *b* et *c* correspondent respectivement aux spectres des figures 5A, 5B et 5C.

La figure 5E montre, au niveau du pixel central du détecteur 20, des courbes représentant l'atténuation linéique spectrale, cette dernière étant respectivement obtenue à partir des ratios

$-ln\left(\frac{S_i}{S_{i,0}}\right)$ (courbe $a$), $-ln\left(\frac{S_i^{p,ref}}{S_{i,0}}\right)$ (courbe $b$) et $-ln\left(\frac{\hat{S}_i^{'p}}{S_{i,0}}\right)$ (courbe $c$). On observe à nouveau l'effet de correction procuré par l'invention : passage du spectre de la courbe $a$ au spectre de la courbe $c$. La courbe de référence, correspondant à l'atténuation spectrale, est la courbe $b$.

L'invention pourra s'appliquer dans des procédés d'imagerie spectrale mettant en œuvre des rayonnements ionisants, en particulier des rayonnements X ou gamma, pour des applications médicales ou plus généralement, dans le contrôle non destructif d'objets, visant à investiguer la structure interne dudit objet. L'objet peut être, par exemple, un bagage, un produit industriel, un élément de structure d'une installation, par exemple une tuyauterie, un déchet nucléaire, ou un composant aéronautique...

L'invention permet une estimation de la composante primaire d'un rayonnement, limitant ainsi l'influence du rayonnement diffusé. On améliore alors la qualité de l'image obtenue, et en particulier la résolution spatiale. La quantification de l'atténuation produite par l'objet est plus précise, ce qui permet une meilleure estimation de la composition de l'objet examiné. Il en résulte des résultats plus précis, et plus conformes à l'objet examiné.

**Revendications**

**1.** Procédé de correction d'une image spectrale formée par un rayonnement électromagnétique ionisant transmis par un objet, l'objet étant disposé entre une source d'irradiation (11) et un détecteur (20), la source d'irradiation étant apte à émettre un rayonnement électromagnétique ionisant, dit rayonnement incident $(I^0)$, vers l'objet ;

- le détecteur (20) comportant des pixels ($20_i$), chaque pixel étant configuré pour détecter un rayonnement transmis par l'objet (10) vers le détecteur, et à en mesurer un spectre ($S_i$), le rayonnement transmis ($I$) comportant un rayonnement diffusé ($I^{diff}$), causé par la diffusion du rayonnement incident $(I^0)$ dans l'objet (10), et un rayonnement primaire ($I^p$) ;
- un masque (15) étant interposé entre la source (11) et l'objet (10), le masque comportant des éléments absorbants ($15_x$), aptes à atténuer une partie du rayonnement incident $(I^0)$, la projection de chaque élément absorbant sur le détecteur formant une zone d'ombre ($20_x$), de telle sorte que le détecteur (20) comporte une pluralité de zones d'ombre espacées les unes des autres, chaque zone d'ombre ($20_x$) comportant au moins un pixel ($20_{i \in 20_x}$) ;

le procédé comportant:

a) irradiation de l'objet et mesure, par les pixels du détecteur ($20_i$), d'un spectre en énergie ($S_i$), représentatif du rayonnement ($I$) transmis par l'objet ;

le procédé étant **caractérisé en ce qu'**il comporte également les étapes suivantes :

b) définition d'un modèle spatial ($mod_K$) du spectre du rayonnement diffusé, de façon à obtenir, en différents pixels du détecteur, une estimation du spectre du rayonnement diffusé ( $\hat{S}_{i,K}^{diff}$ ) détecté par chaque pixel ($20_i$), le modèle spatial étant défini par des paramètres (K) ;

c) prise en compte d'une fonction de coût ( $\mathcal{F}(K)$ ), la fonction de coût étant définie en différents pixels, et prenant en compte, au niveau de chaque pixel, une variation spatiale ( $\nabla\hat{S}_{i,K}^{p\prime}$ ) d'une estimation du spectre du rayonnement primaire transmis par l'objet (10) en l'absence de masque, l'estimation du spectre du rayonnement primaire ( $\hat{S}_{i,K}^{p\prime}$ ) en l'absence de masque étant obtenue en comparant, pour chaque pixel :

• le spectre ($S_i$) mesuré par le pixel lors de l'étape a) ;

• une estimation du spectre du rayonnement diffusé ( $\hat{S}_{i,K}^{diff}$ ) obtenue à l'aide du modèle spatial ($mod_K$) résultant de l'étape b) ;

d) détermination des paramètres *(K)* du modèle spatial ($mod_K$) pour lesquels la fonction de coût ( $\mathcal{F}(K)$ ) est minimale ou maximale ;

e) pour au moins un pixel ($20_i$), estimation du spectre rayonnement diffusé atteignant le pixel ( $\hat{S}_{i}^{diff}$ ), en appliquant les paramètres du modèle spatial *(K)* déterminés lors de l'étape d) ;

f) pour au moins un pixel, correction du spectre ($S_i$) mesuré lors de l'étape a) en utilisant l'estimation du spectre du rayonnement diffusé résultant de l'étape e), de façon à obtenir un spectre corrigé $S_i^*$ ) correspondant à une estimation du spectre du rayonnement primaire ( $\hat{S}_i^p, \widehat{S}_i^{\prime p}$ ) atteignant le pixel.

2. Procédé selon la revendication 1, dans lequel les pixels du détecteur s'étendent selon un plan de détection, et dans lequel le modèle spatial est défini selon deux axes (X, Y) définissant le plan de détection.

3. Procédé selon la revendication 2, dans lequel :

- l'étape a) est mise en œuvre selon une pluralité de configurations, à chaque configuration étant associée une orientation ($\theta$) du détecteur par rapport à l'objet, de façon à mesurer, par chaque pixel, des spectres ($S_{i,\theta}$) correspondant à différentes orientations ;
- le modèle spatial défini lors de l'étape b) prend en compte une variation du spectre du rayonnement diffusé en fonction de l'orientation ($\theta$) du détecteur par rapport à l'objet.

4. Procédé selon l'une quelconque des revendications précédentes, dans lequel lors de l'étape c), l'estimation du spectre primaire ( $\hat{S}_{i,K}^{p\prime}$ ) transmis par l'objet vers chaque pixel, en l'absence de masque (15), est obtenue en appliquant une matrice de correction ($C_i$), définie pour chaque pixel, à une différence entre :

• le spectre ($S_i$) mesuré par le pixel lors de l'étape a) ;

• une estimation du spectre du rayonnement diffusé ( $\hat{S}_{i,K}^{diff}$ ) obtenue à l'aide du modèle paramétrique ($mod_K$) résultant de l'étape b).

5. Procédé selon la revendication 4, dans lequel la matrice de correction ($C_i$) est obtenue au cours d'une phase de calibration, sans objet entre la source d'irradiation et le détecteur, selon les étapes de calibration suivantes :

cal-i) irradiation du détecteur respectivement en interposant et sans interposer le masque entre la source et le détecteur, de façon à obtenir, pour chaque pixel, un spectre de plein flux ( $F_i^0$ ) et un spectre de masque ( $M_i^0$ ); cal-ii) prise en compte d'une fonction paramétrique pour déterminer des termes de la matrice, la fonction paramétrique dépendant de paramètres ;

cal-iii) prise en compte du spectre de plein flux et du spectre de masque pour déterminer les paramètres de la fonction paramétrique, de façon à déterminer les termes de la matrice de correction ;

la matrice de correction ($C_i$) étant triangulaire.

6. Procédé selon l'une quelconque des revendications précédentes, dans lequel le modèle spatial du rayonnement de diffusion est exprimé selon un produit :

    - d'une matrice ($B$) représentative d'une répartition spatiale du rayonnement diffusé ;
    - d'un vecteur de paramètres ($K$) comportant les paramètres du modèle spatial de diffusion ;

de telle sorte que l'étape d) comporte une estimation du vecteur de paramètres (K).

7. Procédé selon l'une quelconque des revendications précédentes, dans lequel l'étape b) comporte une définition d'un modèle spectral du rayonnement diffusé, pour prendre en compte, en chaque pixel, une évolution spectrale du rayonnement diffusé, de telle sorte que le modèle spatial et le modèle spectral sont définis par un vecteur de paramètres (K).

8. Procédé selon la revendication 7, dans lequel le modèle spatial et le modèle spectral du peuvent être exprimés selon un produit :

    - d'une matrice ($B$) représentative d'une répartition spatiale du rayonnement diffusé ;
    - d'une matrice ($\Psi$) représentative de l'évolution spectrale du rayonnement diffusé ;
    - du vecteur de paramètres (K) *;*

de telle sorte que l'étape d) comporte une estimation du vecteur de paramètres (K).

9. Procédé selon l'une quelconque des revendications précédentes, dans lequel chaque élément absorbant ($15_x$) du masque (15) est apte à absorber entre 5 % et 80 % du rayonnement auquel il est exposé, dans une bande d'énergie prédéterminée.

10. Procédé selon l'une quelconque des revendications précédentes, dans lequel l'étape a) comporte une normalisation du spectre du rayonnement transmis par l'objet ($S_i$), mesuré par le pixel, par un spectre ($S_{0,i}$) mesuré par le pixel sans objet disposé entre la source et le détecteur.

11. Procédé selon l'une quelconque des revendications précédentes, dans lequel, le masque (15) s'étendant selon une surface, chaque élément absorbant est distant d'un autre élément absorbant d'une distance inférieure à 1 cm.

12. Support d'enregistrement d'informations, comportant des instructions pour l'exécution des étapes b) à f) du procédé selon l'une quelconque des revendications 1 à 11, à partir des spectres mesurés lors de l'étape a), ces instructions étant aptes à être exécutées par un microprocesseur.

13. Dispositif pour l'acquisition de spectres d'un rayonnement transmis par un objet (10) comportant :

    • une source d'irradiation (11), configurée pour émettre un rayonnement électromagnétique ionisant, dit rayonnement incident ($I^0$), vers ledit objet (10);
    • un détecteur (20) comportant des pixels ($20_i$), chaque pixel étant configuré pour détecter un rayonnement (10) transmis par l'objet vers le détecteur, et à en acquérir un spectre ($S_i$) ;
    • un masque (15), apte à être interposé entre la source (11) et l'objet (10), ledit masque comportant des éléments absorbants ($15_x$), aptes à absorber une partie dudit rayonnement incident ($I^0$), et dont une projection sur le détecteur définit des zones d'ombres (20x) distantes les unes des autres ;
    • un processeur (21), configuré pour recevoir des spectres acquis par chaque pixel, et à mettre en œuvre les étapes b) à f) du procédé objet de l'une quelconque des revendications 1 à 11.

**Patentansprüche**

1. Verfahren zur Korrektur eines Spektralbilds, das von einer ionisierenden elektromagnetischen Strahlung gebildet ist, die von einem Objekt übertragen wird, wobei das Objekt zwischen einer Bestrahlungsquelle (11) und einem Detektor (20) angeordnet ist, wobei die Bestrahlungsquelle geeignet ist, eine ionisierende elektromagnetische Strahlung, die einfallende Strahlung $(I^0)$, zum Objekt hin zu emittieren;

- wobei der Detektor (20) Pixel $(20_i)$ aufweist, jeder Pixel dazu ausgebildet ist, eine Strahlung zu detektieren, die vom Objekt (10) zum Detektor hin übertragen wird, und davon ein Spektrum $(S_i)$ zu messen, wobei die übertragene Strahlung $(I)$ eine Streustrahlung $(I^{diff})$, die von der Streuung der einfallenden Strahlung (1°) im Objekt (10) verursacht wird, und eine Primärstrahlung $(I^p)$ aufweist;
- wobei eine Maske (15) zwischen die Quelle (11) und das Objekt (10) gesetzt ist, wobei die Maske Absorptionselemente $(15_x)$ aufweist, die geeignet sind, einen Anteil der einfallenden Strahlung (1°) zu dämpfen, wobei die Projektion jedes Absorptionselements einen Schattenbereich $(20_x)$ bildet, so dass der Detektor (20) eine Mehrzahl von Schattenbereichen aufweist, die voneinander beabstandet sind, wobei jeder Schattenbereich $(20_x)$ wenigstens einen Pixel $(20_{i \in 20_x})$ aufweist;
das Verfahren aufweisend:

a) Bestrahlen des Objekts und Messen, durch die Pixel des Detektors $(20_i)$, eines Energiespektrums $(S_i)$, das für die Strahlung $(I)$ repräsentativ ist, die vom Objekt übertragen wird;

wobei das Verfahren **dadurch gekennzeichnet ist, dass** es ebenfalls die folgenden Schritte umfasst:

b) Festlegen eines Raummodells $(mod_K)$ des Spektrums der Streustrahlung, um in verschiedenen Pixeln des Detektors eine Schätzung des Spektrums der Streustrahlung ( $\hat{S}_{i.K}^{diff}$ ) zu erhalten, die von jedem Pixel $(20_i)$ detektiert wird, wobei das Raummodell durch Parameter (K) festgelegt wird;
c) Berücksichtigen einer Kostenfunktion (F(K)), wobei die Kostenfunktion in verschiedenen Pixeln festgelegt ist und an jedem Pixel eine räumliche Veränderung ( $\nabla\hat{S}_{i.K}^{p\prime}$ ) einer Schätzung des Spektrums der Primärstrahlung berücksichtigt, das bei Nichtvorhandensein einer Maske vom Objekt (10) übertragen wird, wobei die Schätzung des Spektrums der Primärstrahlung ( $\hat{S}_{i.K}^{p\prime}$ ) bei Nichtvorhandensein einer Maske durch Vergleich von Folgendem für jeden Pixel erhalten wird:

- das Spektrum $(S_i)$, das vom Pixel beim Schritt a) gemessen wird;
- eine Schätzung des Spektrums der Streustrahlung ( $\hat{S}_{i.K}^{diff}$ ), die mithilfe des Raummodells $(mod_K)$ aus Schritt b) erhalten wird;

d) Bestimmen der Parameter (K) des Raummodells $(mod_K)$, für welche die Kostenfunktion (F(K)) minimal oder maximal ist;
e) für wenigstens einen Pixel $(20_i)$, Schätzen des Spektrums der Streustrahlung, die den Pixel erreicht ( $\hat{S}_i^{diff}$ ), unter Anwendung der Parameter des Raummodells (K), die beim Schritt d) bestimmt werden;
f) für wenigstens einen Pixel, Korrigieren des Spektrums $(S_i)$, das beim Schritt a) gemessen wird, unter Nutzung der Schätzung des Spektrums der Streustrahlung, das aus dem Schritt e) hervorgeht, um ein korrigiertes Spektrum ( $S_i^*$ ) zu erhalten, das einer Schätzung des Spektrums der Primärstrahlung ( $\hat{S}_i^p, \widehat{S'}_i^p$ ) entspricht, die den Pixel erreicht.

2. Verfahren nach Anspruch 1, wobei sich die Pixel des Detektors entlang einer Detektionsebene erstrecken und wobei das Raummodell entlang zweier Achsen (X, Y) festgelegt ist, die die Detektionsebene definieren.

3. Verfahren nach Anspruch 2, wobei:

- der Schritt a) gemäß einer Mehrzahl von Konfigurationen durchgeführt wird, wobei jeder Konfiguration eine Ausrichtung $(\theta)$ des Detektors im Verhältnis zum Objekt zugeordnet ist, um durch jeden Pixel Spektren $(S_{i,\theta})$

zu messen, die verschiedenen Ausrichtungen entsprechen;
- das Raummodell, das beim Schritt b) festgelegt wird, eine Veränderung des Spektrums der Streustrahlung in Abhängigkeit von der Ausrichtung ($\theta$) des Detektors im Verhältnis zum Objekt berücksichtigt.

4. Verfahren nach einem der vorhergehenden Ansprüche, wobei beim Schritt c) die Schätzung des Primärspektrums ($\hat{S}_{i.K}^{p'}$), das bei Nichtvorhandensein einer Maske (15) vom Objekt zu jedem Pixel hin übertragen wird, erhalten wird, indem eine Korrekturmatrix ($C_i$), die für jeden Pixel festgelegt ist, auf eine Differenz zwischen Folgendem angewandt wird:

    - dem Spektrum ($S_i$), das vom Pixel beim Schritt a) gemessen wird;
    - einer Schätzung des Spektrums der Streustrahlung ($\hat{S}_{i.K}^{diff}$), die mithilfe des Parametermodells ($mod_K$) aus Schritt b) erhalten wird.

5. Verfahren nach Anspruch 4, wobei die Korrekturmatrix ($C_i$) im Verlauf einer Kalibrierungsphase ohne Objekt zwischen der Bestrahlungsquelle und dem Detektor gemäß den folgenden Kalibrierungsschritten erhalten wird:

    cal-i) Bestrahlen des Detektors unter Zwischensetzen bzw. ohne Zwischensetzen der Maske zwischen die Quelle und den Detektor, um für jeden Pixel ein Vollflussspektrum ($F_i^0$) und ein Maskenspektrum ($M_i^0$)) *zu erhalten;*
    cal-ii) Berücksichtigen einer Parameterfunktion zur Bestimmung der Terme der Matrix, wobei die Parameterfunktion von Parametern abhängt;
    cal-iii) Berücksichtigen des Vollflussspektrums und des Maskenspektrums zur Bestimmung der Parameter der Parameterfunktion, um die Terme der Korrekturmatrix zu bestimmen;

wobei die Korrekturmatrix ($C_i$) dreieckig ist.

6. Verfahren nach einem der vorhergehenden Ansprüche, wobei das Raummodell der Streustrahlung gemäß einem Produkt von Folgendem ausgedrückt wird:

    - einer Matrix ($B$), die für eine räumliche Verteilung der Streustrahlung repräsentativ ist;
    - einem Parametervektor ($K$), der die Parameter des Streuungs-Raummodells aufweist;

so dass der Schritt d) eine Schätzung des Parametervektors *(K) aufweist.*

7. Verfahren nach einem der vorhergehenden Ansprüche, wobei der Schritt b) ein Definieren eines Spektralmodell der Streustrahlung aufweist, um in jedem Pixel eine spektrale Entwicklung der Streustrahlung zu berücksichtigen, so dass das Raummodell und das Spektralmodell von einem Parametervektor ($K$) definiert sind.

8. Verfahren nach Anspruch 7, wobei das Raummodell und das Spektralmodell der gemäß einem Produkt von Folgendem ausgedrückt werden können:

    - einer Matrix ($B$), die für eine räumliche Verteilung der Streustrahlung repräsentativ ist;
    - einer Matrix ($\psi$), die für die spektrale Entwicklung der Streustrahlung repräsentativ ist;
    - des Parametervektors *(K)*;

so dass der Schritt d) eine Schätzung des Parametervektors *(K) aufweist.*

9. Verfahren nach einem der vorhergehenden Ansprüche, wobei jedes Absorptionselement ($15_x$) der Maske (15) geeignet ist, zwischen 5 % und 80 % der Strahlung zu absorbieren, der es in einem vorbestimmten Energieband ausgesetzt ist.

10. Verfahren nach einem der vorhergehenden Ansprüche, wobei der Schritt a) ein Normalisieren des Spektrums der Strahlung, die vom Objekt ($S_i$) übertragen wird, das vom Pixel gemessen wird, durch ein Spektrum *($S_{0,i}$)* aufweist, das ohne Objekt, das zwischen der Quelle und dem Detektor angeordnet ist, vom Pixel gemessen wird.

**11.** Verfahren nach einem der vorhergehenden Ansprüche, wobei, die Maske (15) sich entlang einer Fläche erstreckend, jedes Absorptionselement um einen Abstand kleiner als 1 cm von einem anderen Absorptionselement beabstandet ist.

**12.** Träger zur Speicherung von Informationen mit Anweisungen für die Durchführung der Schritte a) bis f) des Verfahrens nach einem der Ansprüche 1 bis 11 anhand der Spektren, die beim Schritt a) gemessen werden, wobei diese Anweisungen geeignet sind, von einem Mikroprozessor ausgeführt zu werden.

**13.** Vorrichtung für die Erfassung von Spektren einer Strahlung, die von einem Objekt (10) übertragen wird, aufweisend:

- eine Bestrahlungsquelle (11), die dazu ausgebildet ist, eine ionisierende elektromagnetische Strahlung, die einfallende Strahlung ($I^0$), zum Objekt (10) hin zu emittieren;
- einen Detektor (20), der Pixel ($20_i$) aufweist, wobei jeder Pixel dazu ausgebildet ist, eine Strahlung (10) zu detektieren, die vom Objekt zum Detektor hin übertragen wird, und davon ein Spektrum ($S_i$) zu erfassen;
- eine Maske (15), die geeignet ist, zwischen die Quelle (11) und das Objekt (10) gesetzt zu sein, wobei die Maske Absorptionselemente ($15_x$) aufweist, die geeignet sind, einen Anteil der einfallenden Strahlung ($I^0$) zu absorbieren, und wovon eine Projektion auf den Detektor Schattenbereiche (20x) definiert, die voneinander entfernt sind;
- einen Prozessor (21), der dazu ausgebildet ist, Spektren zu empfangen, die von jedem Pixel erfasst werden, und die Schritte b) bis f) des Verfahrens nach einem der Ansprüche 1 bis 11 durchzuführen.

**Claims**

**1.** Method for correcting a spectral image formed by ionizing electromagnetic radiation transmitted by an object, the object being placed between a radiation source (11) and a detector (20), the radiation source being able to emit ionizing electromagnetic radiation, called incident radiation ($I^0$), toward the object;

- the detector (20) comprising pixels ($20_i$), each pixel being configured to detect radiation transmitted by the object (10) to the detector, and to measure a spectrum ($S_i$) thereof, the transmitted radiation ($I$) comprising scattered radiation ($I^{diff}$), caused by scattering of the incident radiation ($I^0$) in the object (10), and primary radiation ($I^p$);
- a mask (15) being interposed between the source (11) and the object (10), the mask comprising absorbent elements ($15_x$), which are able to attenuate one portion of the incident radiation ($I^0$), the projection of each absorbent element onto the detector forming one shadow region ($20_x$), such that the detector (20) comprises a plurality of shadow regions that are spaced apart from one another, each shadow region ($20_x$) comprising at least one pixel ($20_{i \in 20_x}$);

the method comprising:

a) irradiating the object and measuring, by means of the pixels ($20_i$) of the detector, an energy spectrum ($S_i$) representative of the radiation ($I$) transmitted by the object;

the method being **characterized in that** it also comprises the following steps:

b) defining a spatial model ($mod_K$) of the spectrum of the scattered radiation, so as to obtain, for various pixels of the detector, an estimate ($\hat{S}_{i,K}^{diff}$) of the spectrum of the scattered radiation detected by each pixel ($20_i$), the spatial model being defined by parameters (K);

c) taking into account a cost function ($\mathcal{F}(K)$), the cost function being defined for various pixels and taking into account, at the level of each pixel, a spatial variation ($\nabla \hat{S}_{i,K}^{p\prime}$) in an estimate of the spectrum of the primary radiation transmitted by the object (10) in the absence of the mask, the estimate ($\hat{S}_{i,K}^{p\prime}$) of the spectrum of the primary radiation in the absence of the mask being obtained by comparing, for each pixel:

- the spectrum ($S_i$) measured by the pixel in step a);

- an estimate ( $\hat{S}_{i,K}^{diff}$ ) of the spectrum of the scattered radiation, which estimate is obtained using the spatial model ($mod_K$) resulting from step b);

d) determining the parameters *(K),* of the spatial model ($mod_K$), for which the cost function ( $\mathcal{F}(K)$ ) is minimal or maximal;

e) for at least one pixel ($20_i$), estimating the scattered-radiation spectrum reaching the pixel ( $\hat{S}_i^{diff}$ ), by applying the parameters of the spatial model *(K)* that were determined in step d);

f) for at least one pixel, correcting the spectrum ($S_i$) measured in step a) using the estimate of the spectrum of the scattered radiation resulting from step e), so as to obtain a corrected spectrum ( $S_i^*$ ) corresponding to an estimate ( $\hat{S}_i^p, \widehat{S'}_i^p$ ) of the spectrum of the primary radiation reaching the pixel.

2. Method according to Claim 1, wherein the pixels of the detector lie in a detection plane, and wherein the spatial model is defined along two axes (X, Y) defining the detection plane.

3. Method according to Claim 2, wherein:

- step a) is implemented in a plurality of configurations, each configuration being associated with one orientation ($\theta$) of the detector with respect to the object, so as to measure, by means of each pixel, spectra ($S_{i,\theta}$) corresponding to various orientations;
- the spatial model defined in step b) takes into account a variation in the spectrum of the scattered radiation as a function of the orientation ($\theta$) of the detector with respect to the object.

4. Method according to any one of the preceding claims, wherein, in step c), the primary spectrum ( $\hat{S}_{i,K}^{p'}$ ) transmitted by the object to each pixel, in the absence of the mask (15), is estimated by applying a correction matrix ($C_i$), defined for each pixel, to a difference between:

- the spectrum ($S_i$) measured by the pixel in step a);

- an estimate ( $\hat{S}_{i,K}^{diff}$ ) of the spectrum of the scattered radiation, which estimate is obtained using the parametric model ($mod_K$) resulting from step b).

5. Method according to Claim 4, wherein the correction matrix ($C_i$) is obtained in a calibrating phase, with no object between the radiation source and the detector, according to the following calibrating steps:

cal-i) irradiating the detector with and without the mask interposed between the source and the detector, so as to obtain, for each pixel, a full-flux spectrum ( $F_i^0$ ) and a masked spectrum ( $M_i^0$ ), respectively;
cal-ii) taking into account a parametric function in order to determine terms of the matrix, the parametric function depending on parameters;
cal-iii) taking into account the full-flux spectrum and the masked spectrum to determine the parameters of the parametric function, so as to determine the terms of the correction matrix;

the correction matrix ($C_i$) being triangular.

6. Method according to any one of the preceding claims, wherein the spatial model of the scattered radiation is expressed as a product:

- of a matrix (B) representative of a spatial distribution of the scattered radiation;
- of a vector of parameters (K) comprising the parameters of the spatial scattering model;

such that step d) comprises estimating the vector of parameters (K).

7. Method according to any one of the preceding claims, wherein step b) comprises defining a spectral model of the scattered radiation, in order to take into account, in each pixel, a spectral variation of the scattered radiation, such that the spatial model and spectral model are defined by a vector of parameters (K).

8. Method according to Claim 7, wherein the spatial model and the spectral model of the may be expressed as a product:

  - of a matrix (B) representative of a spatial distribution of the scattered radiation;
  - of a matrix ($\Psi$) representative of the spectral variation of the scattered radiation;
  - of the vector of parameters (K);

such that step d) comprises estimating the vector of parameters (K).

9. Method according to any one of the preceding claims, wherein each absorbent element ($15_x$) of the mask (15) is able to absorb between 5% and 80% of the radiation to which it is exposed, in a predetermined energy band.

10. Method according to any one of the preceding claims, wherein step a) comprises normalizing the spectrum of radiation transmitted by the object ($S_i$) and measured by the pixel, by a spectrum ($S_{0,i}$) measured by the pixel with no object placed between the source and the detector.

11. Method according to any one of the preceding claims, wherein, the mask (15) extending over an area, each absorbent element is distant from another absorbent element by a distance of smaller than 1 cm.

12. Data-storage medium, comprising instructions for executing steps b) to f) of the method according to any one of Claims 1 to 11, on the basis of the spectra measured in step a), these instructions being able to be executed by a microprocessor.

13. Device for acquiring spectra of radiation transmitted by an object (10), comprising:

  - a radiation source (11), configured to emit ionizing electromagnetic radiation, called incident radiation (1°), toward said object (10);
  - a detector (20) comprising pixels ($20_i$), each pixel being configured to detect radiation (10) transmitted by the object to the detector, and to acquire a spectrum ($S_i$) thereof;
  - a mask (15), able to be interposed between the source (11) and the object (10), said mask comprising absorbent elements ($15_x$), which are able to absorb a portion of said incident radiation (1°), and a projection of which onto the detector defines shadow regions (20x) that are distant from one another;
  - a processor (21), configured to receive the spectra acquired by each pixel, and to implement steps b) to f) of the method that forms the subject matter of any one of Claims 1 to 11.

**Fig. 1A**

**Fig. 1B**

**Fig. 1C**

**Fig. 2**

**Fig. 3A**

**Fig. 3B**

**Fig. 3C**

**Fig. 4A**

**Fig. 4B**

**Fig. 5A**          **Fig. 5B**          **Fig. 5C**

**Fig. 5D**

**Fig. 5E**

**EP 3 864 624 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3153888 A

**Littérature non-brevet citée dans la description**

- **SOSSIN A.** Fast scattering simulation tool for multi-energy x-ray imaging. *Nuclear Instruments and Methods in Physics Research,* 2015, vol. 802, 60-66